# EUROPÄISCHE PATENTSCHRIFT

(11) **EP 1 601 297 B1**
(45) Veröffentlichungstag und Bekanntmachung des Hinweises auf die Patenterteilung: **17.07.2013**
(21) Anmeldenummer: 03704149.8
(22) Anmeldetag: 07.03.2003
(51) Int. Cl.: A61B 17/72

(54) **VERRIEGELUNGSSCHRAUBE FÜR MARKNAGEL**
LOCKING SCREW FOR AN INTRAMEDULLARY NAIL
VIS DE BLOCAGE POUR CLOU INTRAMEDULLAIRE

(43) Veröffentlichungstag der Anmeldung: 07.12.2005
(60) Teilanmeldung: 10010953.7
(73) Patentinhaber: Synthes GmbH, 4436 Oberdorf (CH)
(72) Erfinder: SENN Peter, CH-4437 Waldenburg (CH); SCHLIENGER, André, CH-4142 Münchenstein (CH); BUETTLER, Markus, CH-4702 Oensingen (CH)
(74) Vertreter: Lusuardi, Werther
(86) Internationale Anmeldenummer: PCT/CH2003/000157
(87) Internationale Veröffentlichungsnummer: WO 2004/078049

(56) Entgegenhaltungen:
- EP-A- 1 214 914
- EP-A1- 1 281 365
- WO-A-03/015649
- US-A- 4 657 001
- US-A- 4 754 749

## Beschreibung

Die Erfindung bezieht sich auf eine Verriegelungsschraube gemäss dem Oberbegriff des Patentanspruchs 1.

Die Verriegelung von Marknägeln gehört zum Stand der Technik. Die Einführung der Verriegelungsschrauben in die Querbohrungen des Marknagels erfolgt entweder mit Hilfe eines bildgebenden Verfahrens (Röntgenkontrolle) oder einer mehr oder weniger komplizierten Zielvorrichtung. In beiden Fällen ist eine gewisse Zielungenauigkeit nicht zu vermeiden, d.h. die Schraubenspitze lässt sich nicht exakt koaxial zur Mittelachse der Querbohrung ausrichten, sondern weicht davon um einen gewissen Betrag ab. Damit die Verriegelungsschraube trotz dieses Zielfehlers in die Querbohrung mündet und durch diese hindurchgebracht werden kann, wird der Aussendurchmesser der Schraube relativ zum Durchmesser der Querbohrung unterdimensioniert. Bleibt die Zielungenauigkeit im Rahmen dieser Unterdimensionierung, so kann die Verriegelungsschraube trotz des Zielfehlers problemlos durch die Querbohrung geführt werden. Allerdings weist nun die Verriegelungsschraube - wegen der Unterdimensionierung - ein gewisses Spiel auf relativ zur Querbohrung.

Dieses Spiel definiert, um welchen Betrag sich die Knochenhauptfragmente, welche mittels Verriegelungsschrauben im entsprechenden Verriegelungsloch fixiert werden, relativ zum Nagel und somit aufgrund der Starrheit des Nagels auch relativ zu anderen mit demselben Nagel befestigten Knochenhauptfragmente bewegen können. Zusammen mit der Flexibilität des Materials und der Gesamtvorrichtung kann dies kumuliert eine Grösse annehmen, welche eine erfolgreiche Heilung verhindert, oder massgeblich verzögert. Um die Anwendbarkeit der Verriegelung für den Chirurgen zu garantieren, ist dieses Spiel zwar unumgänglich, doch ist es klinisch bei gewissen Indikationen (z.B. im Falle von metaphysären Fragmenten) unerwünscht.

Selbst Nägel mit vollem Querschnitt, welche im Verriegelungsloch ein Innengewinde aufweisen können, sind nicht spielfrei. Das Innengewinde verhindert lediglich, dass der Nagel sich axial auf der Verriegelungsschraube bewegen kann.
Eine mehrere Gewindebereiche aufweisende Verriegelungsschraube ist aus der EP 1 214 914 bekannt. Eine weitere verdrehsichere Befestigungsvorrichtung ist aus der EP 1 281 365 bekannt. Aus der US4657001 ist eine durch eine Buchse einer femoralen Stützvorrichtung hindurchführbare Schenkelhalsschraube mit einen Schaft bekannt; die Nuten zur Aufnahme eines longitudinalen Verkeilungselements aufweist.

Hier will die Erfindung Abhilfe schaffen. Der Erfindung liegt die Aufgabe zugrunde, eine Verriegelungsschraube zu schaffen, mit der das vorhandene Spiel zwischen ihr und dem Marknagel eliminiert und die Verriegelungsschraube in der Querbohrung des Marknagels verkeilt werden kann.

Die Erfindung löst die gestellte Aufgabe mit einer Verriegelungsschraube, welche die Merkmale des Anspruchs 1 aufweist.

Die durch die Erfindung erreichten Vorteile sind im wesentlichen darin zu sehen, dass dank der erfindungsgemässen Verriegelungsschraube das Spiel zwischen Marknagel und Verriegelungsschraube eliminiert werden kann. Die Erfindung erlaubt aber auch die Schraube in einer ersten Phase mit Spiel einzubringen und dieses erst nachträglich zu eliminieren. Ein weiterer Vorteil besteht darin, dass trotz der Aufhebung des Spiels die Rotierbarkeit der Verriegelungsschraube nicht blockiert wird. Die Vorteile der Beibehaltung der Rotierbarkeit sind vielfältig:
- die Verriegelungsschraube kann auch im spielreduzierten Zustand ein- oder ausgedreht werden (axiale Freiheit); und
- im Falle eines Versagens der Verriegelungsschraube ist es möglich mit gängigen Techniken die Verriegelungsschraube zu extrahieren (durch Ausschlagen oder durch Herausziehen).

Bei der Erfindung weist der Schraubenschaft eine in Richtung des Durchgangs verlaufende, erste Nut auf, welche vorzugsweise einen teilkreisförmigen oder polygonalen Querschnitt aufweist. Diese Ausführung ist platzsparend, benötigt eine geringere Einpresskraft und garantiert eine gute Führung des Verkeilungselementes.

Darüber hinaus weist der Schraubenschaft eine im wesentlichen parallel zur ersten Nut angeordnete, radial um den Rotationswinkel alpha versetzte, zweite Nut auf, welche tiefer ist als die erste Nut. Der Durchgang im Schraubenkopf ist dabei als Langloch ausgebildet ist, in den beide Nuten münden. Zwischen den beiden Nuten ist vorzegsweise eine von der ersten Nut zur zweiten Nut ansteigende Rampe angebracht.

Der Rotationswinkel alpha kann einen Wert von 40° bis 120°, vorzugsweise von 60° bis 90° aufweisen.

Bei dieser Ausführungsform besteht der Vorteil darin, dass die Verkeilung schneller stattfindet und auch wieder schneller gelöst werden kann. Die Verkeilungskraft wird wesentlich erhöht bei (vergleichbar) geringerem Kraftaufwand, da die Rampe eine Art Übersetzung darstellt. Da die Verkeilungskraft über ein Drehmoment aufgebracht wird benötigt der Anwender nur einen relativ geringen Kraftauswand.

Bei einer weiteren Ausführungsform verjüngt sich die Nut mit zunehmendem Abstand vom Schraubenkopf im Querschnitt, vorzugsweise konisch. Dadurch findet die Verkeilung oder Spielverminderung einerseits schneller statt, anderseits kann sie aber auch wieder schneller gelöst werden.

Bei einer weiteren Ausführungsform weist die Nut einen Winkel zur Längsachse der Verriegelungsschraube auf, der vorzugsweise kleiner als 5° ist.

Die Nut kann eine Tiefe aufweisen, welche zwischen 1% und 50%, vorzugsweise zwischen 2 % bis 20 % des Durchmessers des Schraubenschaftes entspricht.

Zweckmässigerweise wird die erfindungsgemässe Verriegelungsschraube in die Querbohrung eines intramedullären Marknagels eingeführt ist, bei welchem der Durchmesser der Querbohrung grösser ist als der Aussendurchmesser ihres Aussengewindes. Bei Einführung eines longitudinales Verkeilungselement in den Durchgang Verriegelungsschraube erfolgt zwischen dem Schraubenschaft der Verriegelungsschraube und der Innenfläche der Querbohrung des Marknagels eine Verkeilung.

Das longitudinale Verkeilungselement kann ein Draht, vorzugsweise mit einem sich im Querschnitt verjüngenden Ende sein. Zweckmässigerweise besitzt dann der Durchgang der Verriegelungsschraube eine zum Drahtquerschnitt korrespondierende Bohrung.

Alternativ dazu kann das longitudinale Verkeilungselement ein Querschnittsprofil aufweisen, welches zum Querschnittsprofil des Durchgangs korrespondiert und vorzugsweise keilförmig ausgebildet ist.
Der Durchgang kann im wesentlichen kreisförmig ausgebildet sein mit einem Durchmesser von 0,5 - 2,0 mm, vorzugsweise von 0,8 - 1,2 mm.
Das longitudinale Verkeilungselement hat typischerweise eine Länge, welche mindestens zwei Drittel des Schraubenschaftes entspricht.
Das longitudinale Verkeilungselement kann an einem seiner Enden einen Anschlag für den Schraubenkopf aufweisen. Damit wird ein Durchstossen des Verkeilungselementes durch den Durchgang verhindert.

Der Durchgang kann auch mit einem Innengewinde versehen sein. Das longitudinale Verkeilungselement kann dann ebenfalls ein Aussengewinde tragen, welches mit dem Innengewinde des Durchgangs korrespondiert.

Die Erfindung und Weiterbildungen der Erfindung werden im folgenden anhand der teilweise schematischen Darstellungen mehrerer Ausführungsbeispiele noch näher erläutert.

Es zeigen:
Fig. 1 eine perspektivische Ansicht einer Verriegelungsschraube mit einem Durchgang im Schraubenkopf und einem Verriegelungselement;
Fig. 2 einen Längsschnitt durch einen Marknagel in dessen Querbohrung die Verriegelungsschraube nach Fig. 1 eingesetzt ist;
Fig. 3 einen um 90° versetzten Längsschnitt durch den Marknagel nach Fig. 2 im Bereich seiner Querbohrung;
Fig. 4 eine perspektivische Ansicht einer modifizierten Verriegelungsschraube mit einer Bohrung im Schraubenkopf und einer Längsnut im Schraubenschaft;
Fig. 5 eine perspektivische Ansicht der Verriegelungsschraube nach Fig. 4 mit einem im Kopf der Schraube eingeführten longitudinalen Verkeilungselement;
Fig. 6 einen vergrösserten Längsschnitt durch die modifizierte Verriegelungsschraube nach Fig. 4;
Fig. 7 einen Querschnitt durch die modifizierte Verriegelungsschraube nach Fig. 4 im Bereich der Querbohrung des Marknagels;
Fig. 8 eine perspektivische Ansicht einer modifizierten Verriegelungsschraube mit einem schlüsselförmigen Durchgang und einem schlüsselförmigen Verriegelungselement;
Fig. 9 eine perspektivische Ansicht einer modifizierten Verriegelungsschraube mit zwei Nuten;
Fig. 10 einen Querschnitt durch die Verriegelungsschraube nach Fig. 9 in einer ersten, unverkeilten Position; und
Fig. 11 einen Querschnitt durch die Verriegelungsschraube nach Fig. 9 in einer zweiten, verkeilten Position; und
Fig. 12 eine schematische Querschnittdarstellung der Verriegelungsschraube nach Fig. 9 mit der ersten und zweiten Position der Verriegelungsschraube.

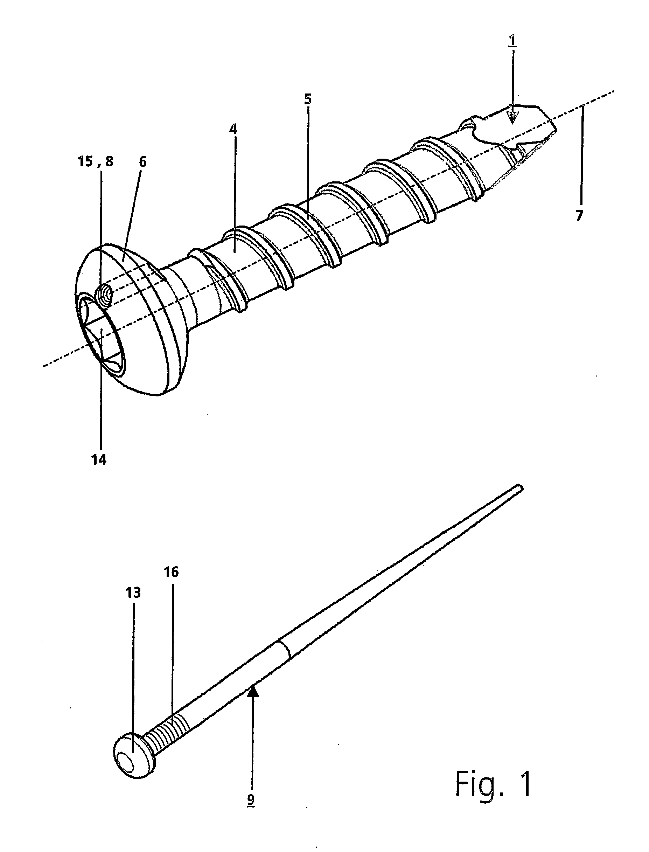

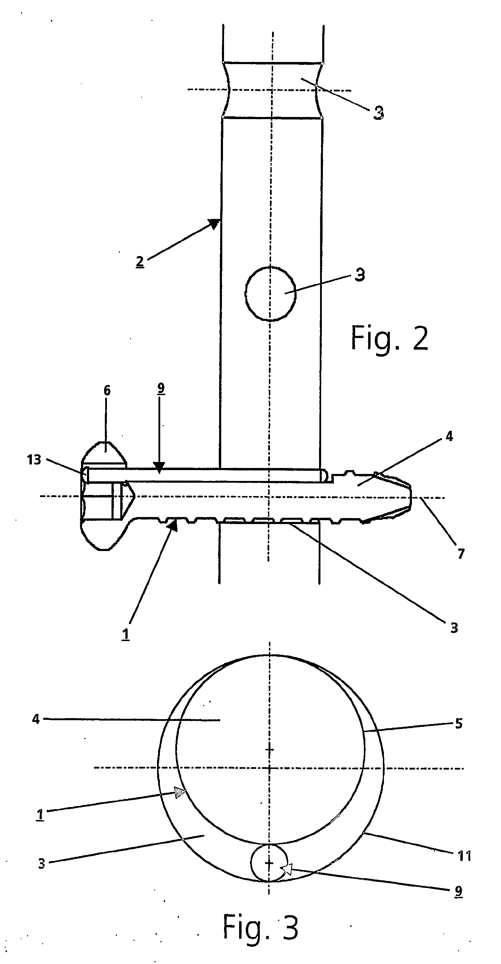

Die in Fig. 1 dargestellte Verriegelungsschraube 1 wird zur Verriegelung eines in Fig. 2 dargestellten, intramedullären Marknagels 2 verwendet, der über mehrere Querbohrungen 3 verfügt. Die Verriegelungsschraube 1 besitzt eine zentrale Längsachse 7 und umfasst einen Schraubenschaft 4, der mit einem Aussengewinde 5 versehen ist sowie einen Schraubenkopf 6 mit einem Innensechskant 14, um die Verriegelungsschraube 1 in eine der Querbohrungen 3 des Marknagels 2 eindrehen zu können.

Der Durchmesser des Schraubenkopfes 6 ist grösser ist als der Aussendurchmesser des Aussengewindes 5 wobei der Schraubenkopf 6 einen im wesentlichen parallel zur Längsachse 7 und zum Aussengewinde 5 verlaufenden Durchgang 8 in Form einer Kreisbohrung mit einem Innengewinde 15 zur Aufnahme eines longitudinalen Verkeilungselementes 9 in Form eines konisch zulaufenden Stiftes mit einem partiellen Aussengewinde 16 und einem kopfseitigen Anschlag 13, wobei das Aussengewinde 16 mit dem Innengewinde 15 korrespondiert. Das Verkeilungselement 9 hat eine Länge welche etwa zwei Drittel der Länge des Schraubenschaftes 4 entspricht.

In Fig. 3 ist dargestellt, wie das Verkeilungselement 9 zwischen der Innenfläche 11 der Querbohrung 3 und dem Aussendurchmesser des Aussengewindes 5 der Verriegelungsschraube 1 verkeilbar ist. Voraussetzung dazu ist der Umstand, dass der Aussendurchmesser des Aussengewindes 5 kleiner ist als der Durchmesser der Querbohrung 3.

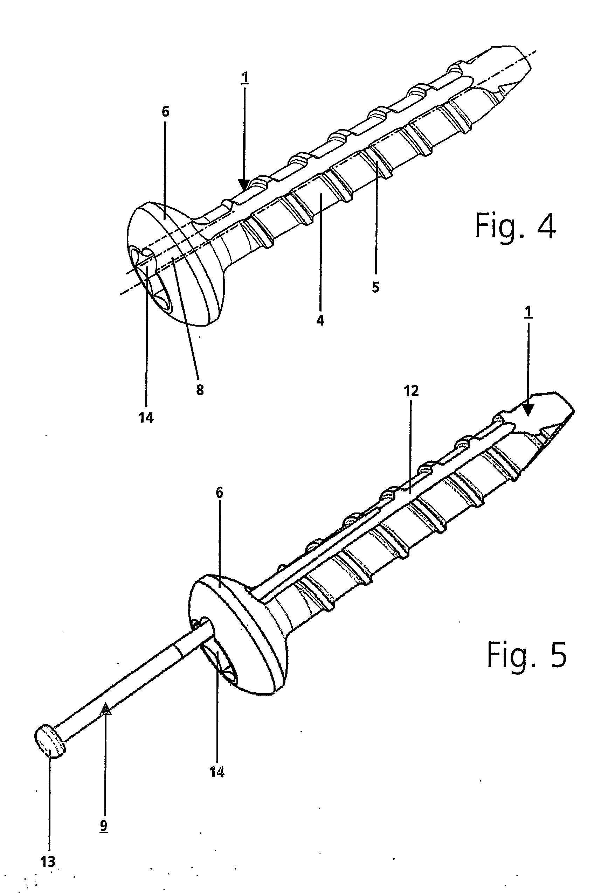

Wie in Fig. 2 und 5 dargestellt weist das longitudinale Verkeilungselement 9 an seinem hinteren Ende einen Anschlag 13 auf, so dass es nicht weiter in den Schraubenkopf 6 hineinstossbar ist. Dadurch wird verhindert, dass das Verkeilungselement 9 in den Markkanal gelangt.

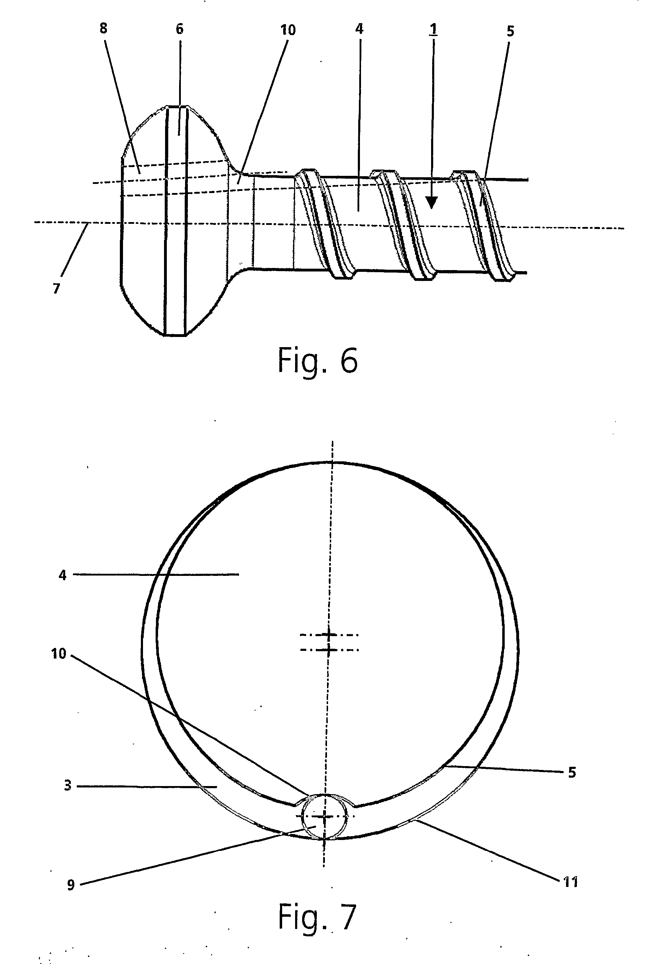

In den Fig. 4 - 7 ist eine Variante der Verriegelungsschraube 1 dargestellt, bei welcher der Schraubenschaft 4 eine mit dem Durchgang 8 (in Form einer Kreisbohrung mit einem Durchmesser von typischerweise 1 mm) fluchtende Nut 10 mit einem teilkreisförmigen Querschnitt aufweist. Die Nut 10 verjüngt sich konisch mit zunehmendem Abstand vom Schraubenkopf 6. Die Nut 10 weist eine Tiefe von 5 % Durchmessers des Schraubenschaftes 4 entspricht. Das ebenfalls konisch zulaufende Verkeilungselement 9 wird bei dieser Variante zwischen der Innenfläche 11 der Querbohrung 3 und der Innenfläche der Nut 10 verkeilt.

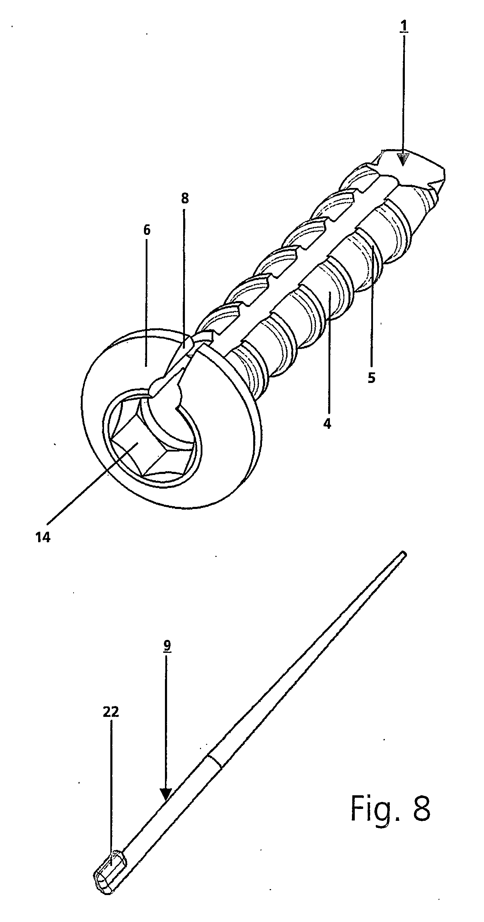

In Fig. 8 ist eine weitere Variante der Verriegelungsschraube 1 dargestellt, bei welcher der Durchgang 8 in Form einer schlüsselförmigen Nut realisiert ist zur Aufnahme eines longitudinalen Verkeilungselementes 9 in Form eines Stabes, welcher an seinem kopfseitigen Ende ein schlüsselförmiges Profil 22 aufweist, welches der schüsselförmigen Nut entspricht. Durch die Form des schlüsselförmigen Profils 22 wird das Verkeilungselement 9 gegen ein Verdrehen relativ zum Durchgang 8 gesichert.

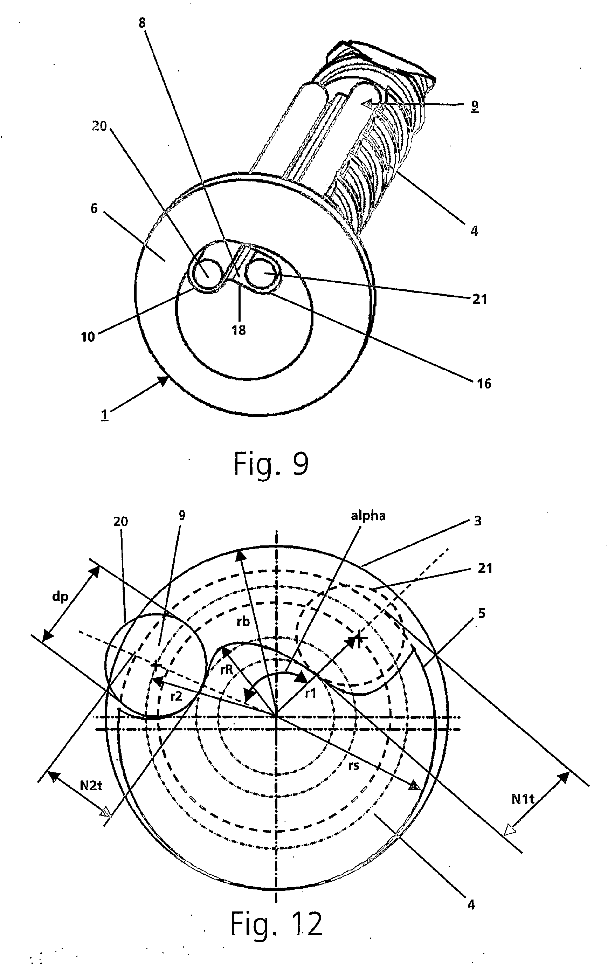

In den Fig. 9 bis 12 ist eine Ausführungsform der Verriegelungsschraube 1 nach der Erfindung dargestellt. Der Schraubenschaft 4 weist bei dieser Variante neben einer ersten Nut 10 eine parallel zur ersten Nut 10 verlaufende, um den Rotationswinkel alpha von 50° radial versetzte, zweite Nut 16 auf, welche tiefer ist als die erste Nut 10. Dabei ist die erste Nut 10 über eine keilförmigen Rampe 18 mit der zweiten Nut 16 verbunden.
Der Durchgang 8 im Schraubenkopf 6 ist dabei als Langloch entsprechend der Geometrie der Rampe 18 ausgebildet, in welches beide Nuten 10,16 münden, so dass das longitudinale Verriegelungselement 9 sowohl in der ersten Nut 10 als auch in der zweiten Nut 16 plaziert werden kann.

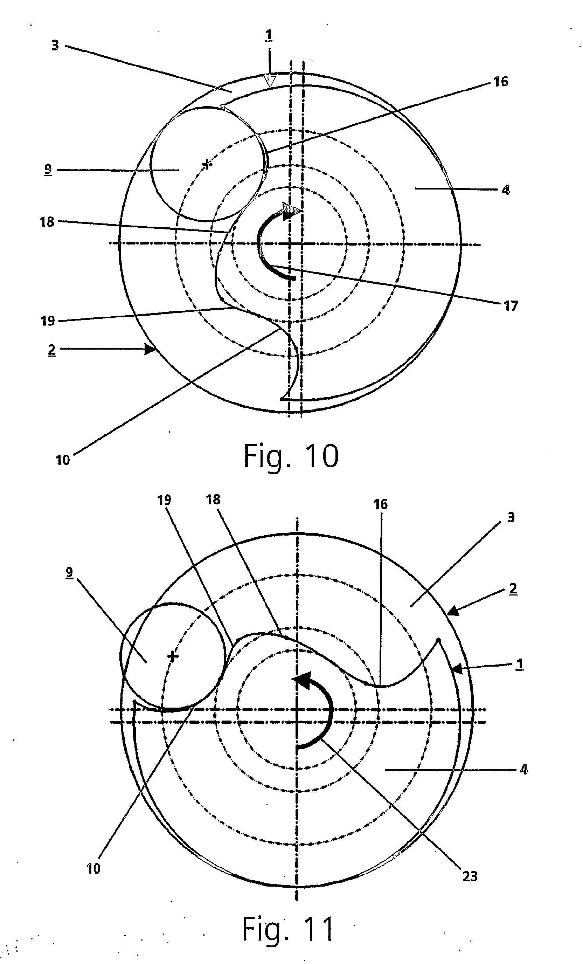

Durch Drehen der Verriegelungsschraube 1 in Richtung des Pfeiles 17 (Uhrzeigersinn) gleitet das in der zweiten Nut 16 liegende longitudinale Verriegelungselement 9 (unverkeilte Position in Fig. 10) entlang der relativ flachen, keilförmigen Rampe 18 bis es in die erste, weniger tiefe Nut 10 fällt (verkeilte Position in Fig. 11). Ein Zurückgleiten des longitudinalen Verriegelungselementes 9 von der ersten Nut 10 in die zweite Nut 16 wird durch die relativ steile Flanke 19 und die Überhöhung relativ zu den beiden Positionen 20 und 21 zwischen den beiden Nuten 10,16 verhindert. Das longitudinale Verriegelungselement 9 bewegt sich dabei im Langloch 8 von der Position 21 zur Position 20 (Fig. 9).

Durch Drehen der Verriegelungsschraube 1 in Richtung des Pfeiles 23 (Gegenuhrzeigersinn) fällt das in der ersten Nut 10 liegende longitudinale Verriegelungselement 9 (verkeilte Position in Fig. 11) wieder in die zweite Nut 16 (unverkeilte Position in Fig. 10), so dass die Verspannung der Verriegelungsschraube 1 wieder aufgehoben ist.

Wie in Fig. 12 dargestellt sollte die Dimensionierung der einzelnen Bauelemente vorteilhafterweise wie folgt sein:
- der Abstand zwischen dem Mittelpunkt des longitudinalen Verriegelungselementes 9 und dem Mittelpunkt des Schraubenschaftes 4 in Position 21 (gestrichelte Linien) beträgt r₁;
- der Abstand zwischen dem Mittelpunkt des longitudinalen Verriegelungselementes 9 und dem Mittelpunkt des Schraubenschaftes 4 in Position 20 (durchgehende Linien) beträgt r₂, wobei r₂ > r₁ ist;
- der Durchmesser des longitudinalen Verriegelungselementes 9 beträgt dp
- der Radius des Aussengewindes 5 des Schraubenschaftes 4 beträgt rₛ
- der Durchmesser der Querbohrung 3 beträgt r_{b} , wobei r_{b} ≧ rₛ
- die erwünschte Presspassung, resp. die Spielreduzierung zwischen dem Aussengewinde 5 des Schraubenschaftes 4, dem longitudinalen Verriegelungselementes 9 und der Querbohrung 3 des Marknagels ergibt sich, wenn die Summe von [(rₛ - N₂ₜ + dₚ) + rₛ)] grösser oder gleich gross ist wie 2 r_{b}, wobei N₂ₜ die Nutentiefe in Position 20 bedeutet.

## Patentansprüche

1. Verriegelungsschraube (1) für einen intramedullären Marknagel (2) mit mindestens einer Querbohrung (3), wobei die Verriegelungsschraube (1) eine zentrale Längsachse (7) aufweist und folgende Elemente umfasst:
A) einen Schraubenschaft (4), der mindestens abschnittsweise mit einem Aussengewinde (5) versehen ist; und
B) einen Schraubenkopf (6), dessen Durchmesser grösser ist als der Aussendurchmesser des Aussengewindes (5),
**dadurch gekennzeichnet, dass**
C) der Schraubenkopf (6) einen im wesentlichen parallel zur Längsachse (7) und zum Aussengewinde (5) verlaufenden Durchgang (8) zur Aufnahme eines longitudinalen Verkeilungselementes (9) aufweist;
D) der Schraubenschaft (4) eine in Richtung des Durchgangs (8) verlaufende, erste Nut (10) aufweist; und
E) der Schraubenschaft (4) eine im wesentlichen parallel zur ersten Nut (10) angeordnete, radial um den Rotationswinkel alpha versetzte, zweite Nut (16) aufweist, welche tiefer ist als die erste Nut (10) und dass der Durchgang (8) im Schraubenkopf (6) als Langloch ausgebildet ist, in den beide Nuten (10,16) münden.

2. Verriegelungsschraube (1) nach Anspruch 1, **dadurch gekennzeichnet, dass** die erste Nut (10) einen teilkreisförmigen oder polygonalen Querschnitt aufweist.

3. Verriegelungsschraube (1) nach Anspruch 1, **dadurch gekennzeichnet, dass** der Durchgang (8) peripher geschlossen ist.

4. Verriegelungsschraube (1) nach Anspruch 3, **dadurch gekennzeichnet, dass** der Rotationswinkel alpha einen Wert von 40° bis 120°, vorzugsweise von 60° bis 90° aufweist.

5. Verriegelungsschraube (1) nach Anspruch 3 oder 4, **dadurch gekennzeichnet, dass** zwischen den beiden Nuten (10,16) eine von der ersten Nut (10) zur zweiten Nut (16) ansteigende Rampe (18) angebracht ist.

6. Verriegelungsschraube (1) nach einem der Ansprüche 1 bis 5, **dadurch gekennzeichnet, dass** sich die Nut (10;16) mit zunehmendem Abstand vom Schraubenkopf (6) im Querschnitt verjüngt, vorzugsweise konisch.

7. Verriegelungsschraube (1) nach einem der Ansprüche 1 bis 6, **dadurch gekennzeichnet, dass** die Nut (10;16) einen Winkel zur Längsachse (7) aufweist, der vorzugsweise kleiner als 5° ist.

8. Verriegelungsschraube (1) nach einem der Ansprüche 1 bis 7, **dadurch gekennzeichnet, dass** der Durchgang (8) im Wesentlichen kreisförmig ausgebildet ist mit einem Durchmesser von 0,5 - 2,0 mm, vorzugsweise von 0,8 -1,2 mm.

9. Verriegelungsschraube (1) nach einem der Ansprüche 2 bis 8, **dadurch gekennzeichnet, dass** die erste Nut (10) eine Tiefe aufweist, welche zwischen 1 % und 50%, vorzugsweise 2 % bis 20 % des Durchmessers des Schraubenschaftes (4) entspricht.

10. Verriegelungsschraube (1) nach einem der Ansprüche 1 bis 9, **dadurch gekennzeichnet, dass** der Durchgang (8) mit einem Innengewinde (15) versehen ist.

11. Vorrichtung umfassend einen intramedullären Marknagel (2) und eine Verriegelungsschraube (1) nach einem der Ansprüche 1 - 10.

12. Vorrichtung nach Anspruch 11, **dadurch gekennzeichnet, dass** die Verriegelungsschraube (1) in eine Querbohrung (3) des intramedullären Marknagels (2) einführber ist und der Aussendurchmesser des Aussengewindes (5) der Verriegelungsschraube kleiner als der Durchmesser der Querbohrung (3) ist.

13. Vorrichtung nach Anspruch 11 oder 12, **dadurch gekennzeichnet, dass** sie zusätzlich ein longitudinales Verkeilungselement (9) umfasst.

14. Vorrichtung nach Anspruch 13, **dadurch gekennzeichnet, dass** das longitudinales Verkeilungselement (9) in den Durchgang (8) der Verriegelungsschraube (1) einführber ist, wobei das longitudinales Verkeilungselement (9) zwischen dem Schraubenschaft (4) der Verriegelungsschraube (1) und der Innenfläche (11) der Querbohrung (3) des Marknagels (2) verkeilbar ist.

15. Vorrichtung nach Anspruch 13 oder 14, **dadurch gekennzeichnet, dass** das longitudinale Verkeilungselement (9) ein Draht ist, vorzugsweise mit einem sich im Querschnitt verjüngenden Ende (12) und der Durchgang (8) der Verriegelungsschraube (1) eine zum Drahtquerschnitt vorzugsweise korrespondierende Bohrung ist.

16. Vorrichtung nach einem der Ansprüche 13 bis 15, **dadurch gekennzeichnet, dass** das longitudinale Verkeilungselement (9) ein Querschnittsprofil aufweist, welches zum Querschnittsprofil des Durchgangs (8) der Verriegelungsschraube (1) korrespondiert und vorzugsweise keilförmig ausgebildet ist.

17. Vorrichtung nach einem der Ansprüche 13 bis 16, **dadurch gekennzeichnet, dass** das longitudinale Verkeilungselement (9) eine Länge hat, welche mindestens zwei Drittel des Schraubenschaftes (4) entspricht.

18. Vorrichtung nach einem der Ansprüche 13 bis 17, **dadurch gekennzeichnet, dass** das longitudinale Verkeilungselement (9) an einem seiner Enden einen Anschlag (13) für den Schraubenkopf (6) aufweist.

19. Vorrichtung nach einem der Ansprüche 13 bis 18, **dadurch gekennzeichnet, dass** die Nutentiefe N₁ₜ derart bemessen ist, dass das longitudinale Verkeilungselement (9) mit einem Spiel relativ zur Querbohrung (3) in die zweite Nut (16) einführbar ist.

20. Vorrichtung nach einem der Ansprüche 13 bis 19, **dadurch gekennzeichnet, dass** das longitudinale Verkeilungselement (9) ein Aussengewinde (16) trägt, welches mit dem Innengewinde (15) des Durchgangs (8) korrespondiert.

## Claims

1. Locking screw (1) for an intramedullary nail (2) with at least one transverse borehole (3), the locking screw (1) having a central longitudinal axis (7) and comprising the following elements:
A) a screw shaft (4) which is provided at least sectionally with an external thread (5); and
B) a screw head (6), the diameter of which is larger than the external diameter of the external thread (5),
**characterized in that**
C) the screw head (6) has a passage (8) for accommodating a longitudinal wedging element (9), the passage (8) extending essentially parallel to the longitudinal axis (7) and to the external thread (5);
D) the screw shaft (4) has a first groove (10) extending in the direction of the passage (8); and
E) the screw shaft (4) has a second groove (16), which is deeper than the first groove (10), the second groove (16) being disposed essentially parallel to the first groove (10) and offset radially by the rotation angle alpha, and that the passage (8) in the screw head (6) is constructed as an elongated hole, in which the two grooves (10, 16) terminate.

2. Locking screw (1) according to claim 1, **characterized in that** the first groove (10) has a partially circular or polygonal cross-section.

3. Locking screw (1) according to claim 1, **characterized in that** the passage (8) is peripherally closed.

4. Locking screw (1) according to claim 3, **characterized in that** the rotation angle alpha has a value of 40° to 120° and preferably of 60° to 90°.

5. Locking screw (1) according to claims 3 or 4, **characterized in that** a ramp (18), rising from the first groove (10) to the second groove (16), is provided between the two grooves (10, 16).

6. Locking screw (1) according to one the claims 1 to 5, **characterized in that** the groove (10; 16) tapers in cross-section, preferably conically, as its distance from the screw head (6) increases.

7. Locking screw (1) according to one of the claims 1 to 6, **characterized in that** the groove (10; 16) makes an angle with the longitudinal axis (7), which preferably is smaller than 5°.

8. Locking screw (1) according to one of the claims 1 to 7, **characterized in that** the passage (8) is essentially circular with a diameter of 0.5-2.0 mm and preferably of 0.8-1.2 mm.

9. Locking screw (1) according to one of the claims 2 to 8, **characterized in that** the first groove (10) has a depth, which corresponds to between 1% and 50% and preferably two 2% to 20% of the diameter of the screw shaft (4).

10. Locking screw (1) according to one of the claims 1 to 9, **characterized in that** the passage (8) is provided with an internal thread (15).

11. Device comprising an intramedullary nail (2) and a locking screw (1) according to one of the claims 1 - 10.

12. Device according to claim 11, **characterized in that** the locking screw (1) is insertable in the transverse borehole (3) of the intramedullary nail (2) and that the external diameter of the external thread (5) of the locking screw is smaller than the diameter of the transverse borehole (3).

13. Device according to claim 11 or 12, further comprising a longitudinal wedging element (9).

14. Device according to claim 13, **characterized in that** the longitudinal wedging element (9) is introducible into the passage (8) of the locking screw (1), wherein the longitudinal wedging element (9) can be wedged between the screw shaft (4) of the locking screw (1) and the inner surface (11) of the transverse borehole (3) of the medullary nail (2).

15. Device according to claim 13 or 14, **characterized in that** the longitudinal wedging element (9) is a wire, preferably with an end (12) tapering in cross-section and the passage (8) is a borehole, preferably corresponding to the cross-section of the wire.

16. Device according to one of the claims 13 to 15, **characterized in that** the longitudinal wedging element (9) has a cross-sectional profile, which corresponds to the cross-sectional profile of the passage (8) of the locking screw (1) and preferably is constructed wedge-shaped.

17. Device according to one of the claims 13 to 16, **characterized in that** the longitudinal wedging element (9) has a length, which corresponds at least to two thirds of the screw shaft (4).

18. Device according to one of the claims 13 to 17, **characterized in that** the longitudinal wedging element (9), at one of its ends, has a stop (13) for the screw head (6).

19. Device according to one of the claims 13 to 18, **characterized in that** the depth of the groove N₁ₜ is such, that the longitudinal wedging element (9) can be introduced into the second groove (16) with clearance relative to the transverse borehole (3).

20. Device according to one of the claims 13 to 19, **characterized in that** the longitudinal wedging element (9) has an external thread (16), which corresponds to the internal thread (15) of the passage (8).

## Revendications

1. Vis de verrouillage (1) pour un clou intramédullaire (2) avec au moins un alésage transversal (3), la vis de verrouillage (1) présentant un axe longitudinal (7) central et comprenant les éléments suivants :
A) une tige de vis (4) qui est munie, au moins par tronçons, d'un filet extérieur (5) ; et
B) une tête de vis (6) dont le diamètre est supérieur au diamètre extérieur du filet extérieur (5),
**caractérisée en ce que**
C) la tête de vis (6) présente une traversée (8) placée essentiellement parallèlement à l'axe longitudinal (7) et au filet extérieur (5) pour la réception d'un élément de clavetage (9) longitudinal ;
D) la tige de vis (4) présente une première rainure (10) placée dans la direction de la traversée (8) ; et
E) la tige de vis (4) présente une deuxième rainure (16), disposée de façon essentiellement parallèle à la première rainure (10), décalée radialement de l'angle de rotation alpha et qui est plus profonde que la première rainure (10), et **en ce que** la traversée (8) dans la tête de vis (6) est constituée en tant que trou oblong dans lequel débouchent les deux rainures (10, 16).

2. Vis de verrouillage (1) selon la revendication 1, **caractérisée en ce que** la première rainure (10) présente une section transversale en forme de portion de cercle ou polygonale.

3. Vis de verrouillage (1) selon la revendication 1, **caractérisée en ce que** la traversée (8) est fermée en périphérie.

4. Vis de verrouillage (1) selon la revendication 3, **caractérisée en ce que** l'angle de rotation alpha présente une valeur de 40° à 120°, de préférence de 60° à 90°.

5. Vis de verrouillage (1) selon la revendication 3 ou 4, **caractérisée en ce que**, entre les deux rainures (10, 16), il est placé une rampe (18) montant à partir de la première rainure (10) vers la deuxième rainure (16).

6. Vis de verrouillage (1) selon une des revendications 1 à 5, **caractérisée en ce que** la rainure (10 ; 16) se rétrécit, de préférence en cône, dans la section transversale quand la distance à la tête de vis (6) augmente.

7. Vis de verrouillage (1) selon une des revendications 1 à 6, **caractérisée en ce que** la rainure (10 ; 16) présente par rapport à l'axe longitudinal (7) un angle qui est de préférence inférieur à 5°.

8. Vis de verrouillage (1) selon une des revendications 1 à 7, **caractérisée en ce que** la traversée (8) est constituée essentiellement de façon circulaire avec un diamètre de 0,5 à 2,0 mm, de préférence de 0,8 à 1,2 mm.

9. Vis de verrouillage (1) selon une des revendications 2 à 8, **caractérisée en ce que** la première rainure (10) présente une profondeur qui correspond à 1 % à 50 %, de préférence à 2 % à 20 %, du diamètre de la tige de vis (4).

10. Vis de verrouillage (1) selon une des revendications 1 à 9, **caractérisée en ce que** la traversée (8) est munie d'un filet intérieur (15).

11. Dispositif comprenant un clou intramédullaire (2) et une vis de verrouillage (1) selon une des revendications 1 - 10.

12. Dispositif selon la revendication 11, **caractérisé en ce que** la vis de verrouillage (1) peut être introduite dans un alésage transversal (3) du clou intramédullaire (2), et **en ce que** le diamètre extérieur du filet extérieur (5) de la vis de verrouillage est inférieur au diamètre de l'alésage transversal (3).

13. Dispositif selon la revendication 11 ou 12, **caractérisé en ce qu'**il comprend en plus un élément de clavetage (9) longitudinal.

14. Dispositif selon la revendication 13, **caractérisé en ce que** l'élément de clavetage (9) longitudinal peut être introduit dans la traversée (8) de la vis de verrouillage (1), l'élément de clavetage (9) longitudinal pouvant être claveté entre la tige de vis (4) de la vis de verrouillage (1) et la surface intérieure (11) de l'alésage transversal (3) du clou intramédullaire (2).

15. Dispositif selon la revendication 13 ou 14, **caractérisé en ce que** l'élément de clavetage (9) longitudinal est un fil métallique, de préférence avec une extrémité (12) se rétrécissant dans la section transversale, et **en ce que** la traversée (8) de la vis de verrouillage (1) est un alésage correspondant de préférence à la section transversale du fil métallique.

16. Dispositif selon une des revendications 13 à 15, **caractérisé en ce que** l'élément de clavetage (9) longitudinal présente un profil de section transversale qui correspond au profil de section transversale de la traversée (8) de la vis de verrouillage (1) et qui est de préférence constitué en forme de coin.

17. Dispositif selon une des revendications 13 à 16, **caractérisé en ce que** l'élément de clavetage (9) longitudinal a une longueur qui correspond au moins aux deux tiers de la tige de vis (4).

18. Dispositif selon une des revendications 13 à 17, **caractérisé en ce que** l'élément de clavetage (9) longitudinal présente à une de ses extrémités une butée (13) pour la tête de vis (6).

19. Dispositif selon une des revendications 13 à 18, **caractérisé en ce que** la profondeur de rainure N₁ₜ est dimensionnée de telle sorte que l'élément de clavetage (9) longitudinal peut être introduit dans la deuxième rainure (16) avec un jeu par rapport à l'alésage transversal (3).

20. Dispositif selon une des revendications 13 à 19, **caractérisé en ce que** l'élément de clavetage (9) longitudinal porte un filet extérieur (16) qui correspond au filet intérieur (15) de la traversée (8).
